# EUROPÄISCHE PATENTANMELDUNG

(11) **EP 2 417 944 A1**
(43) Veröffentlichungstag der Anmeldung: **15.02.2012**
(21) Anmeldenummer: 10172608.1
(22) Anmeldetag: 12.08.2010
(51) Int. Cl.: A61F 5/01

(54) **Unterschenkelorthese**

(71) Anmelder: Ruepp, Thomas, 4132 Muttenz (CH)
(72) Erfinder: Ruepp, Thomas, 4132 Muttenz (CH)
(74) Vertreter: Braun, André jr.

(57) **Zusammenfassung**

Die Erfindung betrifft eine Unterschenkelorthese (1) zur dynamischen Redression von Fussfehlstellungen, mit einem Fussteil (101) und ein einem Unterschenkelteil (103) eines einstückigen Formteils (10), wobei Fussteil (101) und Unterschenkelteil (103) aus einem einstückigen Formteil gebildet sind und wobei die beiden Teile zwecks Schrittausübung zueinander mittels eines Gelenks (105) verbunden und beweglich gelagert sind, wobei die Unterschenkelorthese (1) ein Verstärkungselement (200) aufweist, mittels welchem das Unterschenkelteil (103) und das Fussteil (101) verstärkt sind, wobei sich das Verstärkungselement (200) dorsal wenigsten teilweise entlang des hinteren Oberschenkels und wenigstens teilweise entlang der Fussteils (101) erstreckt.

## Beschreibung

Die Erfindung betrifft eine Unterschenkelorthese gemäss dem Oberbegriff des unabhängigen Patentanspruchs 1.

### Beschreibung

Orthesen sind orthopädische Apparate zur Ruhigstellung, Entlastung, Stabilisierung oder Korrektur von Rumpf oder Gliedmassen, insbesondere von Armen und Beinen.

Unterschenkelorthesen werden insbesondere zur Korrektur bzw. Redression von Varus- bzw. Valgusabweichungen und zur Spitzfusskorrektur verwendet. Bei Instabilität des oberen Sprunggelenks (OSG), des unteren Sprunggelenks (USG) oder Verrenkungen und Verrenkungsbrüchen der Fusswurzel (Chopart, Lisfranc) werden Unterschenkelorthesen zur Korrektur und zum Stützen des Fussgelenks verwendet.

In der Orthopädie bezeichnet der Begriff Redression die unblutige, von Hand oder apparativ ausgeführte Korrektur einer Skelettdeformität mit anschliessender Fixation des betreffenden Skelettabschnittes durch einen Fixationsverband oder eine Orthese. Die Redression erfolgt üblicherweise schrittweise als schonende Etappen-Redression. Redression bezeichnet auch die unblutige Reposition von Knochenbrüchen oder Luxationen durch Schraubenzugapparate.

Um eine Redression eines Fusses zu erreichen wird im Stand der Technik ein Gipsabdruck des zu korrigierenden Fusses erstellt, um anschliessend eine individuell angepasste Unterschenkelorthese herzustellen, was äusserst zeitaufwändig ist. Zudem führen selbst individuell angepasste Unterschenkelorthesen zu Druckstellen auf der Haut.

Die bekannten Unterschenkelorthesen sind üblicherweise als starre oder flexible, auch federnd genannte Orthesen ausgebildet. Flexible Unterschenkelorthesen aus dem Stand der Technik erlauben eine Beweglichkeit des oberen Sprunggelenks (OSG) von 5 - 20 Grad und weisen einen Dorsal- und Ventralanschlag auf.

Basierend auf besagtem Gipsabdruck oder durch elektronisch ermittelte dreidimensionale Abbildungen, wie etwa mittels eines Scanners, wird die Orthese aus formbarem Material hergestellt. Häufig werden Kunststoffe verwendet. In der Patentanmeldung US 2006/200059 A1 wird bspw. ein Verfahren dokumentiert, wobei aus mehreren Lagen Stützschalen durch Laminieren hergestellt werden.

Unterschenkelorthesen weisen üblicherweise mehrere schalenartige Abschnitte auf, wobei der eine Abschnitt der Orthese am Unterschenkel anliegt und wobei ein weiterer Abschnitt am Fuss anliegt. Bei flexiblen Unterschenkelorthesen sind diese beiden Abschnitte mittels eines Gelenks miteinander verbunden. Eine solches Gelenk wird in der Druckschrift DE 19928269 A1 offenbart. Derartige mit einem Gelenk verbundene Abschnitte weisen den Nachteil auf, dass deren Verwindungssteifigkeit nicht genügend gewährleistet ist. Wir bei der Herstellung einer Orthese ein besonderes Augenmerk auf die Verwindungssteifigkeit gelegt, bspw. indem die Materialdicke erhöht wird, so führt dies gleichzeitig zu grösserem Gewicht, was den Tragkomfort reduziert.

Der Erfindung liegt deshalb die Aufgabe zu Grunde, eine Unterschenkelorthese vorzuschlagen, welche die Nachteile des Stands der Technik nicht aufweist. Insbesondere ist es eine Aufgabe der Erfindung, eine kostengünstig herstellbare Unterschenkelorthese vorzuschlagen, welche rationell produziert werden kann.

Erfindungsgemäss wird die Aufgabe gelöst durch eine Unterschenkelorthese zur dynamischen Redression von Gelenkfehlstellungen, mit einem Fussteil und einem Unterschenkelteil, welche mittels eines Gelenks miteinander beweglich verbunden sind, wobei Fussteil und Unterschenkelteil aus einem einstückigen Formteil gebildet sind, wobei ein Verstärkungselement zur Verbesserung der Verwindungssteifigkeit des Formteils der Unterschenkelorthese vorhanden ist.

Einer der Vorteile der Erfindung besteht darin, dass das einstückige Formteil bspw. aus einer einlagigen Platte aus Kunststoff geformt werden kann, welcher mittels Anbringen des Verstärkungselements mindestens teilweise entlang des Unterschenkelteils und des Fussteils die geforderte seitliche Verwindungssteifigkeit gewährt werden kann. Gleichzeitig wird das Gewicht der Unterschenkelorthese nur unwesentlich erhöht. Die Herstellung erfolgt bspw. in thermoplastischer Verarbeitung von einer Platte Polypropylen zur Bildung des einstückigen Formteils, welche mit dem Verstärkungselement aus verbunden wird. Das Verstärkungselement ist vorzugsweise ebenfalls aus Polypropylen gebildet. Es kann eine Materialstärke aufweisen, die gleich, grösser oder kleiner als jene des einstückigen Formteils ist.

Ein anderer Vorteil der Erfindung besteht darin, dass das einstückige Formteil in verschiedenen Grössen herstellbar ist, ähnlich verschiedener Schuhgrössen, wobei die individuelle Anpassung zwecks Redression der Gelenkfehlstellungen mittels eines speziell für den entsprechenden Patienten hergestellten Fussbettes erzielt wird. Dieses Fussbett wird in das Fussteil des Formteils eingesetzt und mit diesem verbunden.

In einer Ausführungsvariante der Erfindung ist das Verstärkungselement der Unterschenkelorthese einteilig ausgebildet ist, wobei das Verstärkungselement dauerhaft mit Unterschenkelteil und Fussteil verbunden ist.

Ein Vorteil der Erfindung besteht darin, dass das Verstärkungselement ähnlich einem langen Streifen oder ähnlich einem flachen Löffel mit Löffelstiel aufweist, wobei das Verstärkungselement entlang des Unterschenkelteils und des Fussteils angeordnet ist. Dabei ist der breitere Teil des Verstärkungselements, welcher einem Löffel ähnlich ist, im Bereich des Fussteils angeordnet. Diese Verstärkung ermöglicht bei Gewährleistung der Flexibilität der Unterschenkelorthese im Bereich des Gelenks eine erhebliche Verbesserung der seitlichen Verwindungssteifigkeit. Die Verbindung erfolgt vorzugsweise durch thermoplastisches Verformen. Die Verbindung kann auch durch Verklebung, Hochfrequenzschweissung oder mittels anderen Verbindungsmitteln, wie etwa Nieten, erfolgen.

In einer anderen Ausführungsvariante der Erfindung erstreckt das Verstärkungselement sich dorsal entlang des Unterschenkelteils erstreckt.

Einer der Vorteile der Erfindung besteht darin, dass das Unterschenkelteil, welches vorzugsweise schalenartig den Unterschenkel mindestens teilweise umfasst, eine Basis bildet, an welcher das Fussteil verwindungssteif angeformt ist.

In einer weiteren Ausführungsvariante der Erfindung ist das Gelenk als integrales Gelenk der Unterschenkelorthese ausgebildet, derart, dass das einstückige Formteil seitliche Ausnehmungen aufweist, welche zwischen Unterschenkelteil und Fussteil angeordnet sind. Das Verstärkungselement bleibt von der Ausnehmung bzw. Einkerbung vorzugsweise unberührt, um das Gelenk nicht zu schwächen.

Einer der Vorteile der Erfindung besteht darin, dass die schmale Ausgestaltung des Verbindungselements, welches mit dem einstückigen Formteil verbunden ist, nach wie vor ermöglicht, mittels Einkerbung an der Unterschenkelorthese im Bereich des Fussgelenks ein scharnierartiges Gelenk auszubilden. Das Verbindungselement verstärkt das Gelenk, wobei gleichzeitig die Beweglichkeit des Fussteils im Verhältnis zum Unterschenkelteil gewährleistet bleibt.

Im Folgenden wird die Erfindung anhand von in den Zeichnungen dargestellten Ausführungsbeispielen näher erläutert. Hierbei gehen aus den Zeichnungen und ihrer Beschreibung weitere wesentliche Merkmale und Vorteile der Erfindung hervor.
Fig. 1 zeigt eine schematische und vereinfacht dargestellte Unterschenkelorthese, welche aus einem einstückigen Formteil gebildet ist;
Fig. 1a zeigt zwei Stellungen der Unterschenkelorthese in unterschiedlichen Neigungswinkeln in einer vereinfachten geschnittenen Darstellung;
Fig. 2 zeigt das Unterschenkelteil der Unterschenkelorthese in einer geschnittenen und abgebrochenen Darstellung im Bereich des Gelenks der Unterschenkelorthese;
Fig. 3 zeigt das Unterschenkelteil der Unterschenkelorthese in einer anderen geschnittenen und abgebrochenen Darstellung in einem Bereich oberhalb des Gelenks der Unterschenkelorthese.

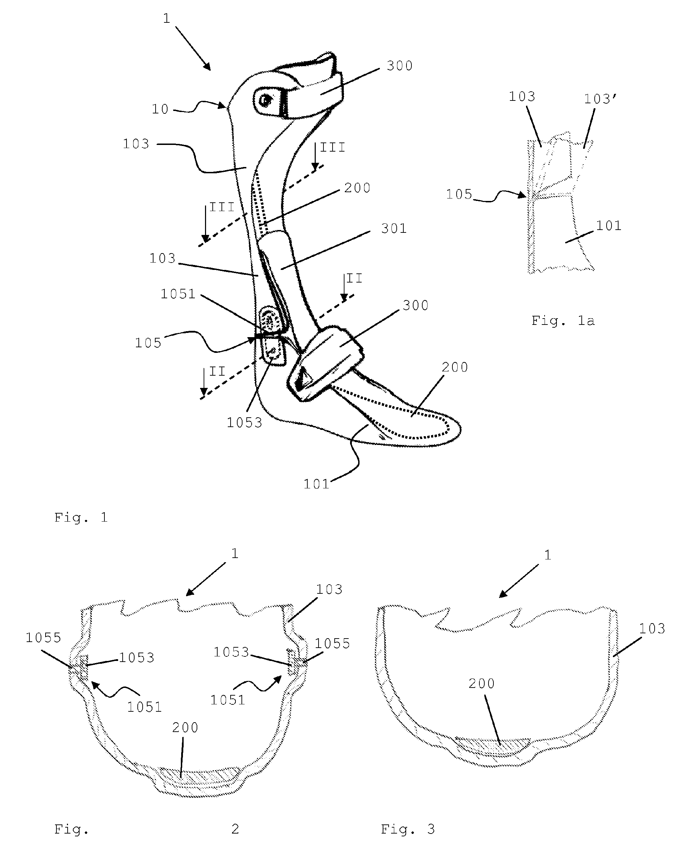

Figur 1 illustriert eine Unterschenkelorthese 1 zur dynamischen Redression von Gelenkfehlstellungen, insbesondere von Fussfehlstellungen, mit einem Fussteil 101 und ein einem Unterschenkelteil 103 eines einstückigen Formteils 10, wobei Fussteil 101 und Unterschenkelteil 103 aus einem einstückigen Formteil gebildet sind und wobei die beiden Teile zwecks Schrittausübung zueinander mittels eines Gelenks 105 verbunden und beweglich gelagert sind. Dabei weist die Unterschenkelorthese 1 ein Verstärkungselement 200 auf, mittels welchem das Unterschenkelteil 103 und das Fussteil 101 verstärkt sind, wobei sich das Verstärkungselement 200 dorsal wenigsten teilweise entlang des hinteren Unterschenkels und wenigstens teilweise entlang der Fussteils 101 erstreckt.

Das Gelenk 105 verbindet schwenkbar bzw. gelenkig aneinander angelenkte Teilbereiche 101,103 des Formteils 10 der Unterschenkelorthese 1.

Figur 2 illustriert das Unterschenkelteil 103 der Unterschenkelorthese 1 in einer geschnittenen und abgebrochenen Darstellung im Bereich des Gelenks 105 der Unterschenkelorthese. Die Unterschenkelorthese 1 weist einen Anschlag bzw. Anschlagmittel auf, welcher eine Dorsalflexion des Unterschenkelteils 103 in Bezug auf das Fussteil 101 beschränkt. Der Anschlag bzw. die Anschlagmittel sind gebildet durch eine im Wesentlichen dreiecksartige Ausnehmung am Formteil 10. Die Stirnflächen der Ausnehmung berühren sich bei einer Vorwärtsneigung des Beins und schränken dadurch die Bewegung des Fussgelenks bewusst ein.

Üblicherweise ist das Unterschenkelteil 103 im Querschnitt im Wesentlichen symmetrisch geformt, so dass ein U-förmiger Querschnitt vorhanden ist. In besonderen, hier nicht illustrierten Fällen kann das Unterschenkelteil 103 asymmetrisch geformt sein, bspw. im Querschnitt im Wesentlichen J-förmig oder L-förmig. Diesfalls würde mit einer einzigen Ausnehmung am Formteil die Gelenkfunktion erzielt.

Figur 1a veranschaulicht zwei Stellungen der Unterschenkelorthese 1 in unterschiedlichen Neigungswinkeln. Es ist zu unterscheiden zwischen einem Anschlag für die Bewegung im Sinne einer Plantarflexion und einer Dorsalflexion eines Fusses. Zur Begrenzung der Plantarflexion - auch Extension genannt - ist ein das Unterschenkelteil 103,103' und das Fussteil 101 verbindendes Sicherungsmittel 1053 vorgesehen, um den Flexionswinkel bei dessen Vergrösserung zu begrenzen. Das Sicherungsmittel 1053 ist bspw. ein aus Metall, Faserverbundstoff oder Kunststoff gebildeter länglicher Streifen oder ein Plättchen, welcher bzw. welches an Unterschenkelteil 103 und Fussteils 101 angeordnet ist und mit diesen beweglich verbunden ist.

Bei der Dorsalflexion - auch Flexion genannt - wird die Ausnehmung am Formteil 10 derart konfiguriert, dass Ausnehmungskanten entstehen, so dass die Ausnehmungskanten des zum Unterschenkelteil 103 beweglich gelagerten Fussteils 101 aufeinander aufstossen.

In dieser Darstellung ist zudem ersichtlich, wie das Verstärkungselement 200 in das Unterschenkelteil 103 des Formteil 10 eingearbeitet ist, so dass eine sich dem Unterschenkel, bzw. der Fessel, angepasste fassende Form ergibt.

Figur 3 illustriert das Unterschenkelteil 103 der Unterschenkelorthese 1 in einer anderen geschnittenen und abgebrochenen Darstellung in einem Bereich oberhalb des Gelenks der Unterschenkelorthese. In dieser Darstellung wird veranschaulicht, wie das Verstärkungselement 200 in das Unterschenkelteil 103 des Formteil 10 eingearbeitet ist, so dass eine sich dem Unterschenkel angepasste fassende Form ergibt.

### Bezugszeichenlegende

- 1: Unterschenkelorthese
- 10: Formteil, einstückig
- 101: Fussteil
- 103: Unterschenkelteil
- 105: Gelenk
- 1051: Ausnehmung seitlich, Einkerbung
- 1053: Sicherungsmittel, Rückstellsicherung
- 1055: Haltemittel, Bolzen, Schraube, Niete
- 200: Verstärkungselement
- 300: Sicherungsmittel, Fixation, Band, Gurt
- 301: Fusseinlage, Sohle

## Patentansprüche

1. Unterschenkelorthese (1) zur dynamischen Redression von Gelenkfehlstellungen, mit einem Fussteil (101) und einem Unterschenkelteil (103) welche mittels eines Gelenks (105) miteinander beweglich verbunden sind, **dadurch gekennzeichnet,**
**dass** Fussteil (101) und Unterschenkelteil (103) aus einem einstückigen Formteil (10) gebildet sind,
**dass** ein Verstärkungselement (200) zur Verbesserung der Verwindungssteifigkeit des Formteils (10) der Unterschenkelorthese (1) vorhanden ist.

2. Unterschenkelorthese (1) gemäss Anspruch 1, **dadurch gekennzeichnet, dass** das Verstärkungselement (200) sich (200) dorsal wenigsten teilweise entlang des einstückigen Formteils (10) und wenigstens teilweise entlang der Fussteils (101) erstreckt.

3. Unterschenkelorthese (1) gemäss einem der Ansprüche 1 oder 2, **dadurch gekennzeichnet, dass** das Verstärkungselement (200) dauerhaft mit Unterschenkelteil (103) und Fussteil (101) verbunden ist.

4. Unterschenkelorthese (1) gemäss einem der Ansprüche 1 bis 3, **dadurch gekennzeichnet, dass** das Verstärkungselement (200) sich dorsal entlang des Unterschenkelteils (103) erstreckt.

5. Unterschenkelorthese (1) gemäss einem der Ansprüche 1 bis 4, **dadurch gekennzeichnet, dass** das Gelenk (105) als integrales Gelenk der Unterschenkelorthese (1) ausgebildet ist, derart, dass das einstückige Formteil (10) seitliche Ausnehmungen (1051) aufweist, welche zwischen Unterschenkelteil (103) und Fussteil (101) angeordnet sind.

6. Unterschenkelorthese (1) gemäss einem der Ansprüche 1 bis 5, **dadurch gekennzeichnet, dass** das Verstärkungselement (200) streifenartig ausgebildet ist.

7. Unterschenkelorthese (1) gemäss einem der Ansprüche 1 bis 6, **dadurch gekennzeichnet, dass** Unterschenkelteil (103) und Fussteil (101) um 5 bis 20 Grad zueinander schwenkbar sind.

8. Unterschenkelorthese (1) gemäss einem der Ansprüche 1 bis 7, **dadurch gekennzeichnet, dass** die Unterschenkelorthese (1) Anschlagmittel zum Beschränken der Bewegung des Unterschenkelteils (103) in Bezug auf das Fussteil (101) aufweist.

9. Unterschenkelorthese (1) gemäss einem der Ansprüche 5 bis 8, **dadurch gekennzeichnet**, die die bzw. jede Ausnehmung (1051) im Wesentlichen dreiecksförmig ausgebildet ist.

10. Unterschenkelorthese (1) gemäss einem der Ansprüche 1 bis 9, **dadurch gekennzeichnet, dass** Formteil (10) und Verbindungselement (200) aus thermoelastischem Material, vorzugsweise Polypropylen, geformt sind, wobei diese mit einander dauerhaft verbunden sind.

11. Unterschenkelorthese (1) gemäss einem der Ansprüche 1 bis 10, **dadurch gekennzeichnet, dass** zur Begrenzung der Plantarflexion ein das Unterschenkelteil (103) und das Fussteil (101) verbindendes Sicherungsmittel (1053) vorhanden ist.
